# EUROPEAN PATENT SPECIFICATION

(11) **EP 3 193 787 B1**
(45) Date of publication and mention of the grant of the patent: **24.10.2018**
(21) Application number: 15742473.0
(22) Date of filing: 13.07.2015
(51) Int. Cl.: A61F 2/32, A61F 2/34

(54) **ACETABULAR PROSTHESIS LINER**
HÜFTPFANNENPROTHESENAUSKLEIDUNG
DOUBLURE DE PROTHÈSE ACÉTABULAIRE

(30) Priority: 15.07.2014 US 201414331842
(43) Date of publication of application: 26.07.2017
(73) Proprietor: Biomet Manufacturing, LLC, Warsaw, IN 46582 (US)
(72) Inventor: SMITH, Aaron P., Warsaw, IN 46582 (US); BAILEY, Kirk J., Rochester, IN 46975 (US)
(74) Representative: Mays, Julie
(86) International application number: PCT/US2015/040140
(87) International publication number: WO 2016/010895

(56) References cited:
- WO-A1-95/01139
- FR-A1- 2 793 137
- US-A- 3 938 198
- US-A- 4 936 861

## Description

### FIELD

The present disclosure relates to an acetabular shell liner, and particularly to a reinforcing structure for a shell liner.

### BACKGROUND

This section provides background information related to the present disclosure which is not necessarily prior art.

Articulating regions of the anatomy can include areas where two bone sections move relative to one another. For example, an acetabulum can provide a region for articulation with a femoral head. The articulating region, however, can become injured, worn, or suffer from a condition such as arthritis, but it can be replaced with various prostheses. Such prostheses can replace the acetabulum, the femoral head, and various other portions of the femur, or other combinations thereof. The replacement of both the acetabulum and the femoral head is generally referred to as a total joint replacement. When the joint being replaced is a hip, the replacement is generally referred to as a total hip replacement.

Surgeons that perform total hip replacements often look to maximize hip joint stability. One way to achieve stability may be with a larger femoral head size. To maximize an acetabular size, however, one generally needs to minimize thickness of the components. This reduction in thickness may lead to deformation of the components during use, insertion, etc. If an acetabular shell liner deforms more than a determined amount of clearance between the head and liner, pinching may occur. US4936861 discloses an acetabular cup prosthesis.

### SUMMARY

The invention concerns an acetabular prosthesis liner as defined in claim 1 and a method of manufacturing an acetabular prosthesis liner as defined in claim 8.

An acetabular prosthesis liner for a hip replacement assembly includes a thin shell liner forming a portion of a sphere and configured to engage an acetabular cup. The thin shell liner includes an outer surface, an inner surface configured to directly engage a primary femoral head an apical portion, an open portion having an open portion thickness measured between the inner surface and the outer surface, wherein a diameter of the inner surface is between 70 % and 99 % of a diameter of the outer surface a centering member on the outer surface a stiffening member extending away from the outer surface of the thin shell liner for an extension distance, the stiffening member formed continuous with the outer surface and the inner surface, the stiffening member being configured to minimise distortion of the thin shell liner wherein the stiffening member has a thickness, and wherein a maximum thickness of the stiffening member is less than the extension distance.

A hip replacement assembly may include an acetabular cup, a large femoral head, a small femoral head. The large femoral head may articulate within the acetabular cup, and the small femoral head is configured to articulatingly fit within the large femoral head. A shell liner forming a portion of a sphere may engage the acetabular cup. The shell liner includes an acetabular cup engaging surface, a large femoral head engaging surface, and a stiffening member integrated with the acetabular cup engaging surface and the large femoral head engaging surface. The stiffening member is further configured to minimize distortion of the shell liner from the portion of the sphere shape when force is applied to the liner. The large femoral head articulatingly engages the shell liner.

A method of manufacturing a thin shell acetabular liner includes forming an outer surface into at least a part of a sphere and forming an inner surface into at least part of a sphere. Forming a flange such that the outer surface includes a first side of the flange, the inner surface includes a second side of the flange opposing the first side of the flange, the flange having a flange thickness defined by the distance between the outer surface and the inner surface, the flange extending a distance away from the outer surface; wherein forming the inner surface and forming the outer surface forms a liner wherein a diameter of the inner surface is between 70 % and 99 % of a diameter of the outer surface wherein the flange thickness is nearly equal to the liner thickness and the flange thickness is smaller than the distance that the flange extends from the outer surface. Furthermore, forming a centering member on the outer surface at a near central position of the outer surface.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
Fig. 1 illustrates an assembled view of a hip replacement including a acetabular shell liner;
Fig 2. illustrates a perspective view of an acetabular shell liner;
Fig. 3 illustrates a cross-sectional view of an acetabular shell liner;
Fig. 3A illustrates a detail cross-sectional view in circle 3A from Fig. 3; and
Fig. 4 illustrates a perspective view of an acetabular shell liner implanted in an acetabular cup.

Corresponding reference numerals indicate corresponding parts throughout the several views of the drawings.

### DETAILED DESCRIPTION

Example embodiments will now be described more fully with reference to the accompanying drawings.

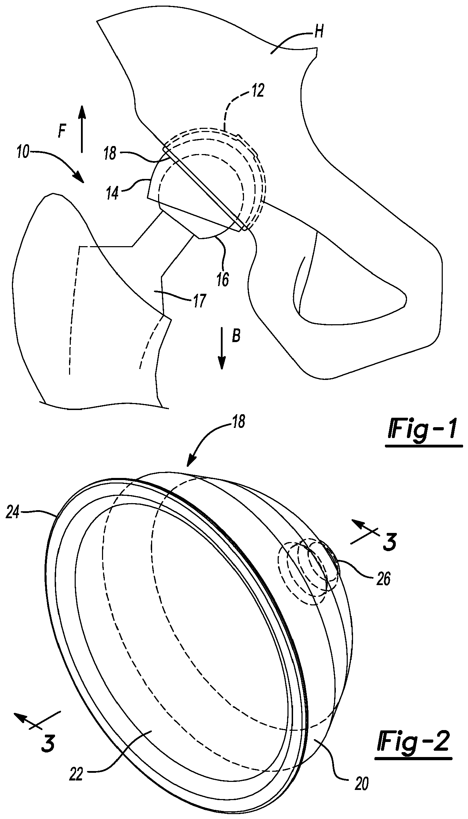

According to the present disclosure and as illustrated in Fig. 1, a hip replacement assembly 10 includes an acetabular cup 12, a large femoral ball 14, a small femoral ball 16, and a shell liner 18. The acetabular prosthesis liner 18 may have a curved inner and outer surface, and further may form a portion of a sphere. The hip replacement assembly 10, for example, may replace a patient's femoral head and acetabulum.

The shell liner 18, as shown in Figs. 2-4, may have a curved outer surface 20 and curved inner surface 22. According to various embodiments, the outer surface 20 and inner surface 22 may form a portion of a sphere, or sphere-like shape. The outer surface 20 may engage the acetabular cup 12. The shell liner 18 may also include conical sections or cylindrical sections. The outer surface 20 may also be an acetabular cup engaging surface and the inner surface 22 may also be a large femoral ball engaging surface. Furthermore, the outer surface 20 may be substantially convex and the inner surface 22 may be substantially concave. The shell liner 18 may also have an apical portion 18a and an open portion 18b. The shell liner 18 further includes a stiffening member or portion 24 that is near the open portion 18b and a centering member 26 that is near the apical portion 18a.

The shell liner 18 may also be thin and have a thickness 25 measured between the inner surface 22 and the outer surface 20. The thickness 25 may be uniform from the apical portion 18a and the open portion 18b. For example, the thickness 25 may be about 2 millimeters (mm) to about 10 mm and further including about 5 mm. The thickness of the liner 18 is generally defined by the thickness 25 of the source material from which the liner 18 is formed. The thickness 25, however, may also vary between an apical portion thickness 25a, measured near the apical portion 18a, and an open portion thickness 25b, measured near the open portion 18b. Thus, the apical portion thickness 25a and the open portion thickness 25b may be unequal. According to various embodiments, the apical thickness may be about 5 mm to about 15 mm and the open portion thickness may be about 2 mm to about 5 mm. The thickness may vary gradually, or the wall of the liner 18 may be stepped.

The shell liner 18 is a thin member. For example, the inner surface 22 of the shell liner 18 may have an inner diameter 22a and the outer surface may have an outer diameter 20a. The shell liner 18 may be thin when the inner diameter 22a has a dimension that is close to the outer diameter 20a. For example, the inner diameter 22a may be about 70% to about 99% of the outer diameter 20a, further including about 85% to about 90 %, and further including about 91% to about 97 %. In various embodiments, the thickness of the liner near the rim may be about 0.5 millimeter (mm) to about 1.5 (mm), including about 1 mm to about 1.5 mm, and further including about 1 mm. The thickness may be the dimension between the inner diameter 22a and the outer diameter 20a.

As a further example, the shell liner 18 may be thin when the inner surface 22 is such that the large femoral head 14 may have a volume that is maximized for the shell liner 18. In various embodiments, the large femoral head 14 with maximum volume may increase a range of motion, reduces the risk of dislocation, and/or minimize point loading on the head 14 compared to the large femoral head 14 at a smaller volume.

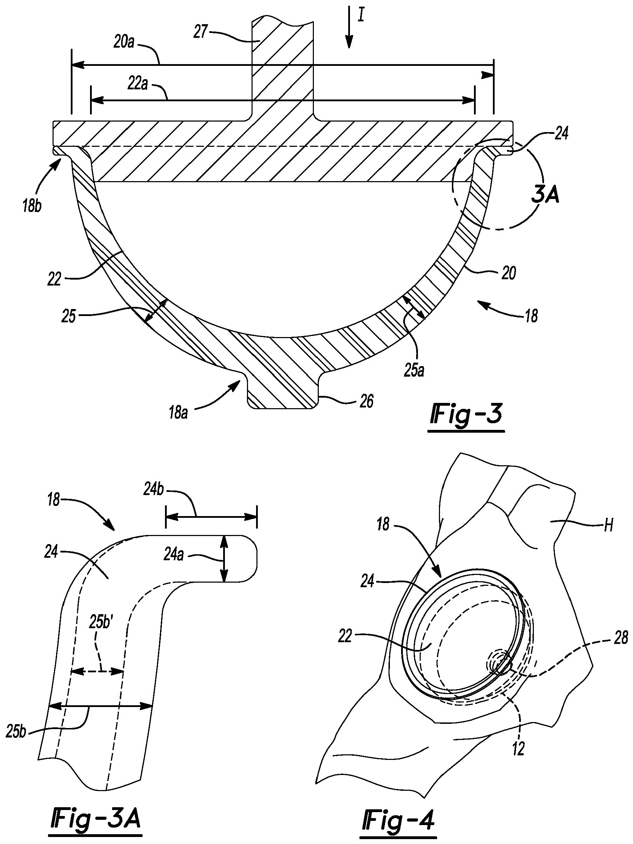

The stiffening member 24 is configured to minimize and/or eliminate distortion of the shell liner 18. In particular, the stiffening member 24 minimizes distortion of the shape or configuration of the liner 18 from the portion of the sphere shape when force, such as a compressive body force, is applied to the shell liner 18. For example, force I may be applied to the shell liner 18 by an insertion tool 27 when the hip replacement assembly 10 is placed in the patient, as shown in Fig. 3 (patient not shown).

In addition, force B may be applied to the outer surface 20 of the liner 18 by a body weight of the patient, for example, when the patient is standing. Therefore, the liner 18 also experiences reaction force F on the inner surface 22 of the liner because of the body weight of the patient. The stiffening member 24 minimizes and /or eliminates distortion of the shell liner 18, despite being thin, when a force is applied to the shell liner.

The stiffening member 24 may extend away from the outer surface 20 of shell liner 18. The stiffening member 24 may be formed as one piece that is integral with the outer surface 20 and the inner surface 22. Therefore, the stiffening member 24 may be a lip or flange. The stiffening member may be formed by machining or bending a solid portion of the liner 18. The stiffening member 24 may also be formed separately from the outer surface 20 and the inner surface 22 and attached to the liner 18. The stiffening member may be attached using commonly known joining methods, such as welding or brazing. The stiffening member 24 may, therefore, have surfaces that are continuous with the outer surface 20 and the inner surface 22.

As shown in Figs. 3 and 3A, the stiffening member 24 may further have a thickness 24a that is smaller than an extension distance 24b of the stiffening member 24. For example, the stiffening member 24 may have the thickness 24a that is about 1 mm in dimension and have the extension 24b that is about 6 mm in dimension. The stiffening member 24 may, however, have the thickness 24a of about 1 mm to about 10 mm. Also, the extension distance 24b of the stiffening member 24 may have a dimension of about 5 mm to about 50 mm.

The stiffening member 24 may also have the thickness 24a similar to the liner thickness 25 or the open portion thickness 25b' (shown in phantom). For example, the liner 18 may have the open portion thickness 25b' near the stiffening member 24 that is similar to the stiffening member thickness 24a. Alternatively, the open portion thickness 25b may be substantially larger than stiffening member thickness 24a. It is understood, however, that the stiffening member 24 may have any appropriate thickness 24a that is generally larger in dimension than the extension 24b.

Again referring to Fig. 1, the hip replacement assembly 10 may include a large femoral head 14 and a small femoral head 16. The large femoral head 14 may be known as a primary head and the small femoral head 16 may be known as a secondary head, which may engage with a femoral stem 17. The small femoral head 16 is configured to be partially fit within the large femoral head 14 and to articulatingly engage the large femoral head 14. For example, the small femoral head 16 may articulate or rotate within the large femoral head 14 in at least one direction according to the movement of the hip H. The large femoral head 14 is configured to articulatingly engage the shell liner 18. For example, the large femoral head 14 may articulate or rotate within the shell liner 18 in at least one direction. Therefore, both the large femoral head 14 and the small femoral ball 16 may articulate or rotate in at least one direction to provide the hip replacement assembly 10 with a range of motion and to reduce the risk of dislocation.

Referring to Figs. 2 and 3, the hip replacement assembly 10 may also include a centering member 26 on the outer surface 20 of the shell liner 18. Specifically, the centering member 26 may be near the apical portion 18a of the shell liner 18. The centering member 26 may be a protrusion that is configured to engage the acetabular cup 12 and center the shell liner 18 within the acetabular cup 12. For example, the centering member 26 may be on the outer surface 20 of the shell liner 18 and configured to engage and center the shell liner 18 within an acetabular cup 12. For example, the centering member 26 may fit within a depression 28 of the acetabular cup 12 such that movement and position of the shell liner 18 relative to the acetabular cup 12 is restricted.

The shell liner 18 may be manufactured or formed by forming the outer surface 20 and the inner surface 22, such as by machining the shell liner 18 from a solid member. One having ordinary skill in the art would understand that forming may also be other methods, such as casting, forging, welding, or 3D printing. Such methods may include investment casting of the liner 18.

In various embodiments, the inner surface 22 and outer surface 20 are each formed into a partial sphere. Forming the inner surface 22 and forming the outer surface 20 also forms the liner thickness 25 of the liner 18. For example, the liner thickness 25 may be formed to be much less than its diameter. As a further example, the liner thickness 25 may be formed to the dimensions as described above.

Furthermore, forming the centering member 26 on the outer surface 20 at a near central position of the outer surface 20. The centering member 26 may be formed as a protrusion that is formed to engage the acetabular cup 12 and center the shell liner 18 within the acetabular cup 12. For example, the centering member 26 may be formed on the outer surface 20 of the shell liner 18 and formed to frictionally engage and center the shell liner 18 within an acetabular cup 12. As a further example, the centering member 26 may be formed to fit within a depression 28 of the acetabular cup 12 such that movement and position of the shell liner 18 relative to the acetabular cup 12 is restricted.

In various embodiments, the stiffening member 24 is formed to extend away from the outer surface 20 to minimize distortion of the shell liner 18. In particular, the stiffening member 24 is formed to minimize distortion from the formed shape, such as the portion of the sphere or sphere-like shape, when pressure is applied to the shell liner 18. The stiffening member 24 may be formed with a thickness that is nearly equal to the liner thickness. For example, the lip may be formed to have dimensions as described above.

The flange, or stiffening member 24, may further be formed to have a thickness that is much smaller than a width of the flange. For example, the stiffening member 24 may be formed to have the dimensions as described above. Forming the stiffening member 24 includes forming the outer surface 20 to include a side of the stiffening member 24 and forming the inner surface 22 to include an opposing side of the stiffening member 24. For example, a cylinder of appropriate material may be machined to form the stiffening member 24 to extend away from the outer surface 20. Further, the liner 18 may be formed and the stiffening member 24 may be machined separately as an annular member and affixed to the liner 18 near the open end 18b.

The shell liner 18 according to various embodiments may provide for a shell acetabular prosthesis liner 18 that maximizes femoral head size for increased hip H joint stability. To avoid deformation of the shell liner 18 bone when under a force, such as a compressive force or pressure, while being inserted in the acetabulum of the hip H, the shell liner 18 includes the stiffening member 24 that is configured to minimize distortion. Therefore, the acetabular shell liner 18 according to the present teachings increases range of motion and reduces the risk of dislocation of the hip replacement assembly 10.

The foregoing description of the acetabular shell liner has been provided for purposes of illustration and description. It is not intended to be exhaustive or to limit the disclosure.

## Claims

1. An acetabular prosthesis liner (18) for a hip replacement assembly comprising:
a thin shell liner having a formed shape configured to engage an acetabular cup (12),
the thin shell liner including:
an outer surface (20);
an inner surface (22) configured to directly engage a primary femoral head (14);
an apical portion (18a);
an open portion (18b) having an open portion thickness (25b) measured between the inner surface and the outer surface, wherein a diameter of the inner surface is between 70 % and 99 % of a diameter of the outer surface ;
a centering member (26) on the outer surface; and
a stiffening member (24) extending away from the outer surface of the thin shell liner for an extension distance (24b), the stiffening member formed continuous with the outer surface and the inner surface, the stiffening member being configured to minimise distortion of the thin shell liner;
wherein the stiffening member has a thickness (24a), and wherein a maximum thickness of the stiffening member is less than the extension distance.

2. The acetabular prosthesis liner of Claim 1, wherein the extension distance is between 5 mm and 50 mm and the thickness of the stiffening member is between 1 mm and 10 mm.

3. The acetabular prosthesis liner of Claim 1 or Claim 2, wherein a ratio of the maximum thickness of the stiffening member to the open portion thickness is approximately unity.

4. The acetabular prosthesis liner of any of Claims 1-3, wherein the centering member is configured to engage the acetabular cup and center the thin shell liner within the acetabular cup.

5. The acetabular prosthesis liner of Claim 4, further comprising:
the acetabular cup;
wherein the centering member includes a protrusion (26) configured to engage a feature of the acetabular cup.

6. The acetabular prosthesis liner of any of Claims 1-5, further comprising:
the primary femoral head;
wherein the primary femoral head articulatingly engages the inner surface of the thin shell liner.

7. The acetabular prosthesis liner of Claim 6, wherein a secondary head articulatingly engages with the primary femoral head; the secondary head further engaging with a femoral stem.

8. A method of manufacturing an acetabular prosthesis liner for a hip replacement assembly, the method comprising:
forming an outer surface of the acetabular prosthesis liner as a partial sphere; forming an inner surface of the acetabular prosthesis liner as a partial sphere; and
forming a flange such that the outer surface includes a first side of the flange, the inner surface includes a second side of the flange opposing the first side of the flange, the flange having a flange thickness defined by the distance between the outer surface and the inner surface, the flange extending a distance away from the outer surface;
and forming a centering member on the outer surface;
wherein forming the inner surface and forming the outer surface forms a liner wherein a diameter of the inner surface is between 70 % and 99 % of a diameter of the outer surface;
wherein the flange thickness is substantially equal to the liner thickness and the flange thickness is smaller than the distance that the flange extends from the outer surface.

9. The method of Claim 8, wherein forming includes at least one of casting, machining, forging, welding, or 3D printing.

10. The acetabular prosthesis liner of Claim 1, wherein the maximum thickness of the stiffening member is 1 mm.

11. The acetabular prosthesis liner of Claim 1, wherein the stiffening member is configured to minimise distortion of the shell liner from the formed shape when a force is applied to the shell liner.

12. The acetabular prosthesis liner of Claim 11, further comprising:
the primary femoral head;
wherein the primary femoral head is configured to apply the force to the shell liner.

13. The acetabular prosthesis liner of any of claims 1-7, wherein the open portion thickness (25b) is between 2 mm and 5 mm.

14. The acetabular prosthesis liner of any of claims 1-7, wherein the open portion thickness (25b) is between 0.5 mm and 1.5 mm.

15. The method of claim 8, wherein a thickness measured between the inner surface and the outer surface is between 0.5 mm and 1.5 mm.

## Patentansprüche

1. Acetabulum-Prothesen-Liner (18) für eine Hüftersatzanordnung, der Folgendes beinhaltet:
einen dünnen Schalen-Liner, der eine geformte Form aufweist, die konfiguriert ist, eine Acetabulumpfanne (12) in Eingriff zu nehmen, wobei der dünne Schalen-Liner Folgendes umfasst:
eine Außenfläche (20);
eine Innenfläche (22), die konfiguriert ist, einen primären Femurkopf (14) direkt in Eingriff zu nehmen;
einen apikalen Abschnitt (18a);
einen offenen Abschnitt (18b), der eine zwischen der Innenfläche und der Außenfläche gemessene Dicke (25b) des offenen Abschnitts aufweist, wobei ein Durchmesser der Innenfläche zwischen 70 % und 99 % eines Durchmessers der Außenfläche beträgt;
ein Zentrierglied (26) auf der Außenfläche; und
ein Versteifungsglied (24), das sich von der Außenfläche des dünnen Schalen-Liners weg über eine Erstreckungsdistanz (24b) erstreckt, wobei das Versteifungsglied kontinuierlich mit der Außenfläche und der Innenfläche gebildet ist, wobei das Versteifungsglied konfiguriert ist, eine Verformung des dünnen Schalen-Liners zu minimieren;
wobei das Versteifungsglied eine Dicke (24a) aufweist und wobei eine maximale Dicke des Versteifungsglieds weniger als die Erstreckungsdistanz ist.

2. Acetabulum-Prothesen-Liner gemäß Anspruch 1, wobei die Erstreckungsdistanz zwischen 5 mm und 50 mm liegt und die Dicke des Versteifungsglieds zwischen 1 mm und 10 mm liegt.

3. Acetabulum-Prothesen-Liner gemäß Anspruch 1 oder Anspruch 2, wobei ein Verhältnis der maximalen Dicke des Versteifungsglieds zur Dicke des offenen Abschnitts ungefähr Eins ist.

4. Acetabulum-Prothesen-Liner gemäß einem der Ansprüche 1-3, wobei das Zentrierglied konfiguriert ist, die Acetabulumpfanne in Eingriff zu nehmen und den dünnen Schalen-Liner innerhalb der Acetabulumpfanne zu zentrieren.

5. Acetabulum-Prothesen-Liner gemäß Anspruch 4, der ferner Folgendes beinhaltet:
die Acetabulumpfanne;
wobei das Zentrierglied einen Vorsprung (26) umfasst, der konfiguriert ist, ein Merkmal der Acetabulumpfanne in Eingriff zu nehmen.

6. Acetabulum-Prothesen-Liner gemäß einem der Ansprüche 1-5, der ferner Folgendes beinhaltet:
den primären Femurkopf;
wobei der primäre Femurkopf die Innenfläche des dünnen Schalen-Liners gelenkig in Eingriff nimmt.

7. Acetabulum-Prothesen-Liner gemäß Anspruch 6, wobei ein zweiter Kopf mit dem primären Femurkopf gelenkig im Eingriff steht; wobei der zweite Kopf ferner mit einem Femurschaft einen Eingriff bildet.

8. Verfahren zum Fertigen eines Acetabulum-Prothesen-Liners für eine Hüftersatzanordnung, wobei das Verfahren Folgendes beinhaltet:
Bilden einer Außenfläche des Acetabulum-Prothesen-Liners als eine Teilkugel; Bilden einer Innenfläche des Acetabulum-Prothesen-Liners als eine Teilkugel; und
Bilden eines Flansches, sodass die Außenfläche eine erste Seite des Flansches umfasst, die Innenfläche eine zweite Seite des Flansches umfasst, die der ersten Seite des Flansches gegenüberliegt, wobei der Flansch eine Flanschdicke aufweist, die durch die Distanz zwischen der Außenfläche und der Innenfläche definiert ist, wobei sich der Flansch um eine Distanz von der Außenfläche weg erstreckt; und Bilden eines Zentrierglieds auf der Außenfläche;
wobei das Bilden der Innenfläche und der Außenfläche einen Liner bildet, wobei ein Durchmesser der Innenfläche zwischen 70 % und 99 % eines Durchmessers der Außenfläche beträgt;
wobei die Flanschdicke im Wesentlichen gleich der Liner-Dicke ist und die Flanschdicke kleiner als die Distanz ist, um die sich der Flansch von der Außenfläche erstreckt.

9. Verfahren gemäß Anspruch 8, wobei das Bilden mindestens eines von Gießen, Maschinen, Schmieden, Schweißen oder 3D-Drucken umfasst.

10. Acetabulum-Prothesen-Liner gemäß Anspruch 1, wobei die maximale Dicke des Versteifungsglieds 1 mm beträgt.

11. Acetabulum-Prothesen-Liner gemäß Anspruch 1, wobei das Versteifungsglied konfiguriert ist, eine Verformung des dünnen Schalen-Liners von der geformten Form zu minimieren, wenn am Schalen-Liner eine Kraft angewandt wird.

12. Acetabulum-Prothesen-Liner gemäß Anspruch 11, der ferner Folgendes beinhaltet:
den primären Femurkopf;
wobei der primäre Femurkopf konfiguriert ist, die Kraft am Schalen-Liner anzuwenden.

13. Acetabulum-Prothesen-Liner gemäß einem der Ansprüche 1-7, wobei die Dicke (25b) des offenen Abschnitts zwischen 2 mm und 5 mm liegt.

14. Acetabulum-Prothesen-Liner gemäß einem der Ansprüche 1-7, wobei die Dicke (25b) des offenen Abschnitts zwischen 0,5 mm und 1,5 mm liegt.

15. Verfahren gemäß Anspruch 8, wobei eine zwischen der Innenfläche und der Außenfläche gemessene Dicke zwischen 0,5 mm und 1,5 mm liegt.

## Revendications

1. Insert de prothèse acétabulaire (18) pour un ensemble de remplacement de hanche comprenant :
un insert de coque fine possédant une forme profilée configurée pour mettre en prise une cupule acétabulaire (12), l'insert de coque fine incluant :
une surface externe (20) ;
une surface interne (22) configurée pour venir directement en prise avec une tête fémorale primaire (14) ;
une partie apicale (18a) ;
une partie ouverte (18b) possédant une épaisseur de partie ouverte (25b) mesurée entre la surface interne et la surface externe, dans lequel un diamètre de la surface interne est entre 70 % et 99 % d'un diamètre de la surface externe ; un élément de centrage (26) sur la surface externe ; et
un élément de rigidification (24) s'écartant de la surface externe de l'insert de coque fine sur une distance d'écartement (24b), l'élément de rigidification formé en continu avec la surface externe et la surface interne, l'élément de rigidification étant configuré pour minimiser une distorsion de l'insert de coque fine ;
dans lequel l'élément de rigidification possède une épaisseur (24a), et dans lequel une épaisseur maximum de l'élément de rigidification est inférieure à la distance d'écartement.

2. Insert de prothèse acétabulaire
selon la revendication 1, dans lequel la distance d'écartement est entre 5 mm et 50 mm et l'épaisseur de l'élément de rigidification est entre 1 mm et 10 mm.

3. Insert de prothèse acétabulaire
selon la revendication 1 ou 2, dans lequel un ratio de l'épaisseur maximum de l'élément de rigidification par rapport à l'épaisseur de la partie ouverte est environ une unité.

4. Insert de prothèse acétabulaire
selon l'une quelconque des revendications 1 à 3, dans lequel l'élément de centrage est configuré pour mettre en prise la cupule acétabulaire et centrer l'insert de coque fine à l'intérieur de la cupule acétabulaire.

5. Insert de prothèse acétabulaire selon la revendication 4, comprenant en outre : la cupule acétabulaire ;
dans lequel l'élément de centrage inclut une protubérance (26) configurée pour mettre en prise une caractéristique de la cupule acétabulaire.

6. Insert de prothèse acétabulaire selon l'une quelconque des revendications 1 à 5, comprenant en outre : la tête fémorale primaire ;
dans lequel la tête fémorale primaire met en prise de manière articulée la surface interne de l'insert de coque fine.

7. Insert de prothèse acétabulaire
selon la revendication 6, dans lequel une tête secondaire met en prise de manière articulée la tête fémorale primaire ; la tête secondaire venant en outre en prise avec une tige fémorale.

8. Procédé de fabrication d'un insert de prothèse acétabulaire pour un ensemble de remplacement de hanche, le procédé comprenant :
la formation d'une surface externe de l'insert de prothèse acétabulaire ; la formation d'une surface interne de l'insert de prothèse acétabulaire comme une sphère partielle ; et
la formation d'une bride de manière à ce que la surface externe inclue un premier côté de la bride, la surface interne inclue un deuxième côté de la bride opposé au premier côté de la bride, la bride ayant une épaisseur de bride définie par la distance entre la surface externe et la surface interne, la bride s'écartant d'une distance depuis la surface externe ;
et la formation d'un élément de centrage sur la surface externe ;
dans lequel la formation de la surface interne et la formation de la surface externe forment un insert dans lequel un diamètre de la surface interne est entre 70 % et 99 % d'un diamètre de la surface externe ;
dans lequel l'épaisseur de la bride est sensiblement égale à l'épaisseur de l'insert et l'épaisseur de la bride est inférieure à la distance sur laquelle la bride s'écarte de la surface externe.

9. Procédé selon la revendication 8, dans lequel la formation inclut au moins l'un parmi la coulée, l'usinage, le forgeage, la soudure ou l'impression 3D.

10. Insert de prothèse acétabulaire selon la revendication 1, dans lequel l'épaisseur maximum de l'élément de rigidification est 1 mm.

11. Insert de prothèse acétabulaire selon la revendication 1, dans lequel l'élément de rigidification est configuré pour minimiser une distorsion de l'insert de coque depuis la forme profilée quand une force est appliquée à l'insert de coque.

12. Insert de prothèse acétabulaire selon la revendication 11, comprenant en outre : la tête fémorale primaire ;
dans lequel la tête fémorale primaire est configurée pour appliquer la force à l'insert de coque.

13. Insert de prothèse acétabulaire selon l'une quelconque des revendications 1 à 7, dans lequel l'épaisseur de la partie ouverte (25b) est entre 2 mm et 5 mm.

14. Insert de prothèse acétabulaire selon l'une quelconque des revendications 1 à 7, dans lequel l'épaisseur de la partie ouverte (25b) est entre 0,5 mm et 1,5 mm.

15. Procédé selon la revendication 8, dans lequel une épaisseur mesurée entre la surface interne et la surface externe est entre 0,5 mm et 1,5 mm.
